# EUROPEAN PATENT APPLICATION

(11) **EP 4 592 294 A1**
(43) Date of publication of application: **30.07.2025**
(21) Application number: 23867386.7
(22) Date of filing: 14.09.2023
(51) Int. Cl.: C07D 495/04, A61K 31/496, A61K 31/4365, A61P 7/02, A61P 9/10

(54) **NOVEL CARBAMATE COMPOUND AND USE THEREOF**

(30) Priority: 22.09.2022 CN 202211159008
(71) Applicant: Chengdu Shibeikang Biomedical Technology Co., Ltd., Chengdu, Sichuan 611730 (CN)
(72) Inventor: ZENG, Yanqun, Chengdu, Sichuan 611730 (CN); ZHOU, Guanglin, Chengdu, Sichuan 611730 (CN); FU, Haixia, Chengdu, Sichuan 611730 (CN); MOU, Xia, Chengdu, Sichuan 611730 (CN); ZHOU, Ning, Chengdu, Sichuan 611730 (CN)
(74) Representative: Kröncke, Rolf
(86) International application number: PCT/CN2023/118773
(87) International publication number: WO 2024/061097

(57) **Abstract**

Provided are an anti-platelet aggregation medicament and use thereof. The medicament is a compound having a structure of formula I, or a pharmaceutically acceptable salt thereof, a solvate thereof, or a deuterated substance thereof. The compound has a remarkable anti-platelet aggregation effect, relatively suitable water solubility and good stability, and thus is expected to become a new type of injectable anti-platelet aggregation medicament.

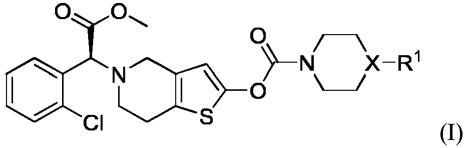

## Description

### FIELD

The present disclosure relates to the field of pharmaceutical chemistry, in particular to a carbamate compound and use of the compound in the manufacture of a medicament for antiplatelet aggregation.

This application claims the priority of Chinese Patent Application No. 202211159008.3, filed with the China National Intellectual Property Administration on September 22, 2022, and titled "NOVEL CARBAMATE COMPOUND AND USE THEREOF", the disclosure of which is hereby incorporated by reference in its entirety.

### BACKGROUND

It is well known in the art that platelet aggregation can trigger a range of diseases related to cardiovascular, cerebrovascular, and other arterial circulatory disorders, including (1) acute coronary syndromes (ACS), such as unstable angina (UA), acute ST-segment elevation myocardial infarction (STEMI), and acute non-ST-segment elevation myocardial infarction (NSTEMI); (2) atherosclerotic diseases, such as myocardial infarction, ischaemic stroke, and peripheral arterial disease; and (3) thrombotic complications. Among the current antiplatelet aggregation drugs, clopidogrel has attracted much attention due to its low risk of bleeding, but the presence of "clopidogrel resistance" phenomenon has greatly limited its onset time of action and application scenarios, for example, limited application in acute thrombosis. Existing technologies have also attempted to prepare injectable dosage forms of clopidogrel, such as ASD-002 nanoemulsion formulation (Ascendia), MDCO-157 injection (encapsulated in cyclodextrin) (CyDex), and JIN-2013 nanoliposome injection (Jina Pharmaceuticals), in order to achieve rapid onset of action and overcome the shortcomings of slow onset of action in the acute therapeutic setting. However, so far, these attempts have all failed due to the solubility, and stability against moisture, light and heat of clopidogrel. In addition, although the 2-Oxo-clopidogrel, an intermediate metabolite of clopidogrel, developed by Chengdu Shibeikang Biomedical Technology Co., Ltd as the leader has overcome the undesired "clopidogrel resistance" reaction and has the advantages such as rapid onset of action and higher bioavailability, it has been found in studies that the 2-Oxo-clopidogrel still has the defect of instability in water, leading to great challenges and limitations to the development of 2-Oxo-clopidogrel into an injectable dosage form. Therefore, the further development of derivatives of the intermediate metabolite 2-Oxo-clopidogrel has attracted extensive attention from all walks of life.

Currently, only vicagrel among the 2-Oxo-clopidogrel-based prodrug molecules has entered the clinical stage, which is in a tablet form. However, in comparative studies, it has been found that vicagrel still has many limitations, such as unsatisfactory solubility and thermal stability, which will likewise largely limit its dosage form and clinical application scenarios.

In conclusion, it is an urgent problem to be solved at present in clinical to develop a prodrug molecule that can sustainably release the active metabolite ingredient (H4) of clopidogrel in the body for a long time and has good water solubility and stability, and to also address the non-injectable problem of clopidogrel.

### SUMMARY

An objective of the present disclosure is to provide a prodrug molecular compound of 2-Oxo-clopidogrel with good stability and excellent solubility in water.

Another objective of the present disclosure is to provide use of the prodrug molecular compound for preventing and/or treating a cardiovascular, cerebrovascular or other arterial circulatory disease due to platelet aggregation.

The present disclosure provides the following technical solutions.

The present disclosure provides a compound represented by Formula (I), or a pharmaceutically acceptable salt, solvate or deuteride thereof: 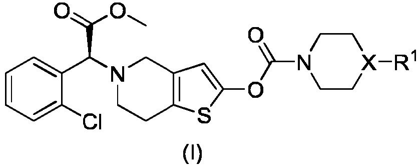 wherein,
X is selected from the group consisting of C or N, and
R¹ is selected from the group consisting of substituted or unsubstituted alkyl, substituted or unsubstituted cycloalkyl, or substituted or unsubstituted N-containing heterocyclyl.

Further, in the compound represented by Formula (I) above, or the pharmaceutically acceptable salt, solvate or deuteride thereof,
X is selected from the group consisting of C or N, and
R¹ is selected from the group consisting of substituted or unsubstituted C₁ to C₆ alkyl, substituted or unsubstituted C₃ to C₆ cycloalkyl, or substituted or unsubstituted C₂ to C₆ N-containing heterocyclyl.

Further, in the compound represented by Formula (I) above, or the pharmaceutically acceptable salt, solvate or deuteride thereof,
X is N,
R¹ is selected from the group consisting of substituted or unsubstituted C₁ to C₆ alkyl, or substituted or unsubstituted C₃ to C₆ cycloalkyl; preferably, R¹ is selected from the group consisting of methyl, ethyl, propyl, cyclopropyl, cyclopentyl or cyclohexyl.

Further, in the compound represented by Formula (I) above, or the pharmaceutically acceptable salt, solvate or deuteride thereof,
XisC,
R¹ is substituted or unsubstituted C₂ to C₆ N-containing heterocyclyl; preferably, R¹ is selected from the group consisting of piperazinyl, piperidinyl or pyrrolyl.

Further, for the compound represented by Formula (I) above, or the pharmaceutically acceptable salt, solvate or deuteride thereof, the compound includes

| Numbe r | Structure | Chemical Name |
|---|---|---|
| 1 | 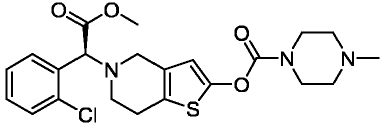 | (S)-5-(1-(2-chlorophenyl)-2-methoxy-2-oxy ethyl)-4,5,6,7-tetrahydrothieno[3,2-c]pyridin -2-yl 4-methylpiperazine-1-carboxylate |
| 2 | 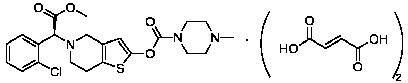 | (S)-5-(1-(2-chlorophenyl)-2-methoxy-2-oxy ethyl)-4,5,6,7-tetrahydrothieno[3,2-c]pyridin -2-yl 4-methylpiperazine-1-carboxylate fu marate |
| 3 | 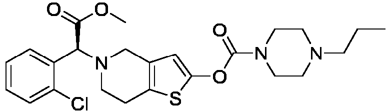 | (S)-5-(1-(2-chlorophenyl)-2-methoxy-2-oxy ethyl)-4,5,6,7-tetrahydrothieno[3,2-c]pyridin -2-yl-4-propylpiperazine-1-carboxylate |
| 4 | 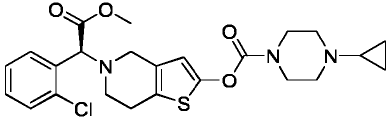 | (S)-5-(1-(2-chlorophenyl)-2-methoxy-2-oxy ethyl)-4,5,6,7-tetrahydrothieno[3,2-c]pyridin -2-yl 4-cyclopropylpiperazine-1-carboxylate |
| 5 | 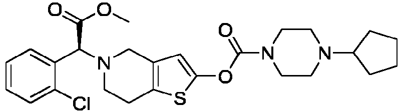 | (S)-5-(1-(2-chlorophenyl)-2-methoxy-2-oxy ethyl)-4,5,6,7-tetrahydrothieno[3,2-c]pyridin -2-yl 4-cyclopentylpiperazine-1-carboxylate |
| 6 | 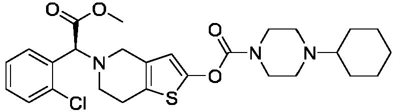 | (S)-5-(1-(2-chlorophenyl)-2-methoxy-2-oxy ethyl)-4,5,6,7-tetrahydrothieno[3,2-c]pyridin -2-yl 4-cyclohexylpiperazine-1-carboxylate |
| 7 | 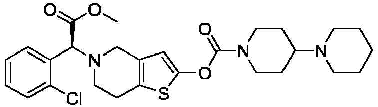 | (S)-5-(1-(2-chlorophenyl)-2-methoxy-2-oxy ethyl)-4,5,6,7-tetrahydrothieno[3,2-c]pyridin -2-yl[1,4'-bipiperidine]-1'-carboxylate |
| 8 | 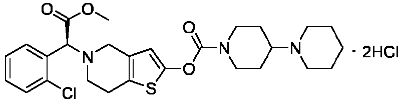 | (S)-5-(1-(2-chlorophenyl)-2-methoxy-2-oxy ethyl)-4,5,6,7-tetrahydrothieno[3,2-c]pyridin -2-yl[1,4'-bipiperidine]-1'-carboxylate hydrochloride |
| 9 | 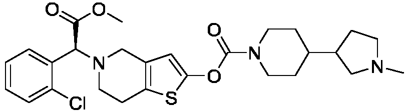 | 5-((S)-1-(2-chlorophenyl)-2-methoxy-2-oxy ethyl)-4,5,6,7-tetrahydrothieno[3,2-c]pyridin -2-yl-4-(1 -methylpyrrolidine-3 -yl)piperidine -1-carboxylate |
| 10 | 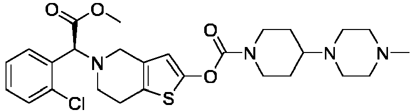 | (S)-5-(1-(2-chlorophenyl)-2-methoxy-2-oxy ethyl)-4,5,6,7-tetrahydrothieno[3,2-c]pyridin -2-yl-4-(4-methylpiperazine-1-yl)piperidine-1-carboxylate |

Further, for the compound above, or the pharmaceutically acceptable salt, solvate or deuteride thereof, the pharmaceutically acceptable salt includes, but is not limited to, fumarate, acetate, ascorbate, benzoate, benzenesulfonate, citrate, hydrochloride, hydrobromide, maleate, mesylate, sulfate, hydrosulfate, nitrate, oxalate, phosphate or succinate.

Further, for the compound above, or the pharmaceutically acceptable salt, solvate or deuteride thereof, the compound or the pharmaceutically acceptable salt thereof is selected from the group consisting of: 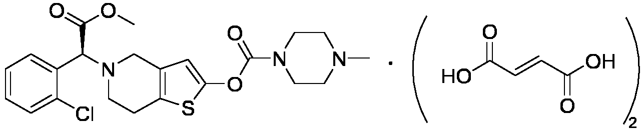 or

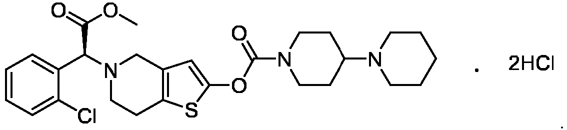

The present disclosure further provides a method for preparing any one of the compounds described above, or the pharmaceutically acceptable salt, solvate or deuteride thereof, comprising: 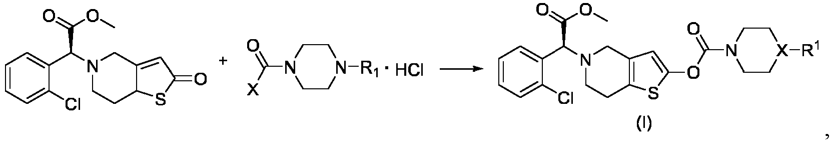 wherein R¹ is defined as any one of corresponding definitions described above, and X is halogen.

The present disclosure further provides use of the compound above, or the pharmaceutically acceptable salt, solvate or deuteride thereof in the manufacture of a medicament for preventing and/or treating a cardiovascular, cerebrovascular or other arterial circulatory disease due to platelet aggregation.

Further, the disease related to cardiovascular, cerebrovascular or other arterial circulatory disorders due to platelet aggregation above includes, but is not limited to, acute coronary artery syndrome, atherosclerotic disease or thrombotic complications.

Further, the acute coronary artery syndrome above includes, but is not limited to, angina pectoris or myocardial infarction.

Still further, the acute coronary artery syndrome above includes, but is not limited to, unstable angina (UA), acute ST-segment elevation myocardial infarction (STEMI) or acute non-ST-segment elevation myocardial infarction (NSTEMI).

Further, the atherosclerotic disease above includes, but is not limited to, myocardial infarction, ischemic stroke or peripheral arterial disease.

Still further, the ischemic stroke above includes, but is not limited to, cerebral stroke.

Further, the thrombotic complications above includes, but is not limited to, pulmonary infarction.

The term "halogen" used herein refers to fluorine, chlorine, bromine or iodine.

The term "C₁ to C₆ alkyl" used herein refers to a straight or branched monovalent alkyl group with a number of carbon atoms from 1 to 6.

### Beneficial effects

The compound of the present disclosure has good efficacy *in vitro* and great pharmacokinetic characteristics. Specifically, the compound of the present disclosure has strong anti-platelet aggregation effect, fast onset of action, high blood drug concentration in pharmacokinetics *in vivo,* high bioavailability, long half-life and good efficacy. In addition, the compound of the present disclosure has good solubility *in vitro,* high stability, low risk of hemolysis, no cardiac toxicity, low risk of vascular irritation and high safety, and is more conducive to being prepared into injections to address clinical deficiencies.

### BRIEF DESCRIPTION OF DRAWINGS

FIG. 1 shows the concentration-effect curve of the compound 2 on hERG channel current.
FIG. 2 is a schematic diagram showing the results of the hemolysis assay of the compound 2 low concentration group (0.5 mg/ml).
FIG. 3 is a schematic diagram showing the results of the hemolysis assay of the compound 2 high concentration group (2.5 mg/ml).
FIG. 4 is a schematic diagram showing the results of the vascular irritation assay of the compound 2 with a low concentration of 0.5mg/ml.
FIG. 5 is a schematic diagram showing the results of the vascular irritation assay of the compound 2 with a high concentration of 1.5mg/ml.
FIG. 6 shows the comparison results of the appearance and characteristics of the comparative example 2 on Day 0 and after being placed under high temperature (60°C) for two days.

### DETAILED DESCRIPTION

Hereinafter the present disclosure will be described in further details in conjunction with examples and test examples. The examples and test examples of the present disclosure are only used to illustrate the technical solutions of the present disclosure, and not to limit the present disclosure. Any equivalent replacements in the art in accordance with the disclosure of the present disclosure are within the protection scope of the present disclosure.

The compounds of the present disclosure, and stereoisomers or pharmaceutically acceptable salts thereof can be prepared by the synthetic routes of the examples, and the conventional conditions of the reaction materials and reaction solvents can be adjusted according to the requirements for substituents or the salt formation, which are achievable by those skilled in the art on the basis of the disclosure of the present disclosure. In addition, the column chromatography used in the present disclosure refers to silica gel column chromatography unless otherwise specifically indicated, and the elution solvent can be determined as a single or mixed elution solvent by considering the reaction solvent and the common knowledge of or means commonly used by those skilled in the art when not specifically stated.

Structures of the compounds were determined by nuclear magnetic resonance (¹H NMR) or liquid chromatograph-mass spectrometry (LC-MS).

Agilent G6120B (used in connection with a liquid phase chromatograph system Agilent 1260) was used for liquid chromatograph-mass spectrometry (LC-MS). Bruker AVANCE-400 or Bruker AVANCE-800 was used for nuclear magnetic resonance (¹H NMR), and the ¹H NMR shift (δ) is presented in a unit of parts per million (ppm), with DMSO-d₆ or CDCl₃ as the detection solvent, and tetramethylsilane (TMS) as the internal standard. Chemical shift is presented in a unit of 10⁻⁶ (ppm).

The term "room temperature" used herein refers to a temperature between 10°C and 25°C.

### Example 1: Preparation of (S)-5-(1-(2-chlorophenyl)-2-methoxy-2-oxyethyl)-4,5,6,7-tetrahydrothieno[3,2-c]pyridin-2-yl 4-methylpiperazine-1-carboxylate (compound 1)

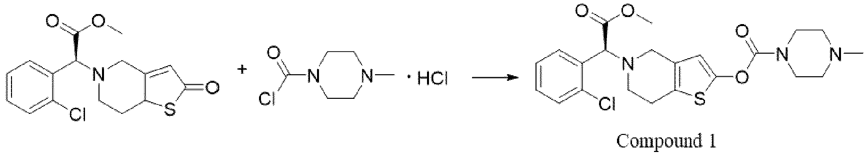

To a 50 ml three-neck flask, methyl (2S)-2-(2-oxo-7,7a-dihydrothieno[3,2-c]pyridin -5(2H,4H,6H)-yl)-2-(2-chlorophenyl)-acetate (500 mg, 1.48 mmol), 4-methylpiperazine-1-for myl chloride hydrochloride (442 mg, 2.22 mmol) and DMF (10 ml) were added. The mi xture was cooled to 0°C with stirring, and dropwise added with DBU (685 mg, 4.5 mmo l). After the dropwise addition was completed, the reaction was performed at room tempe rature for 2 h. After the reaction was completed, the reaction system was added with EA, washed with water twice and saline once. The organic phase was dried over anhydrous Na₂SO₄ and concentrated. The resulting concentrate was separated and purified through ch romatographic column (MeOH:DCM=2:100), and the product was collected, concentrated a nd dried to obtain 580 mg of the title compound as a yellow oil, with a yield of 72.8% and a purity of 96.19%.
ESI-MS: m/z = 464.1(M+H)⁺
¹HNMR (400 MHz, DMSO-d6) δ: 7.60-7.54 (m, 1H), 7.51-7.46 (m, 1H), 7.46-7.35 (m, 2H), 6.39 (s, 1H), 4.86 (s, 1H), 3.67 (s, 3H), 3.57 (s, 2H), 3.40 (s, 2H), 2.80(qt, 2H), 2.63 (d, 2H), 2.48-2.39 (m, 6H), 2.34 (s, 3H)

### Example 2: Preparation of (S)-5-(1-(2-chlorophenyl)-2-methoxy-2-oxyethyl)-4,5,6,7-tetrahydrothieno[3,2-c]pyridin-2-yl 4-methylpiperazine-1-carboxylate fumarate (compound 2)

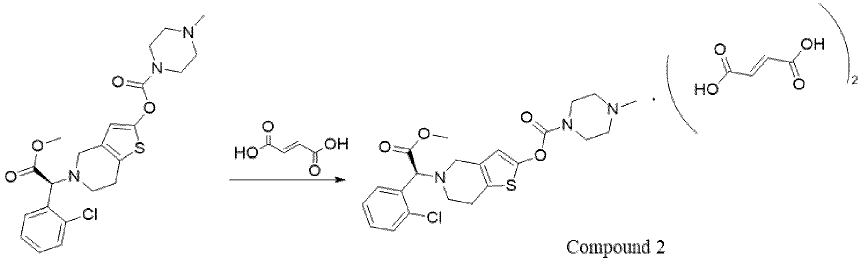

(S)-5-(1-(2-chlorophenyl)-2-methoxy-2-oxyethyl)-4,5,6,7-tetrahydrothieno[3,2-c]pyridi n-2-yl 4-methylpiperazine-1-carboxylate (220 mg, 0.47 mmol) was added to a 25 ml flask, then dissolved with EA (2.5 ml), and added with fumaric acid (110 mg, 0.95 mmol). The mixture was stirred, and a large amount of solid precipitated. The stirring was carried out for 3 h, and the reaction was completed. The reaction system was filtered and dried to obtain 192 mg of the title compound, with a yield of 58.7% and a purity of 98.39%.
ESI-MS: m/z = 464.1(M+H)⁺
¹HNMR (400 MHz, DMSO-d6) δ: 13.82-9.71 (br, 4H), 7.64-7.56 (m, 1H), 7.56-7.47 (m, 1H), 7.46-7.35 (m, 2H), 6.62 (s, 4H), 6.39 (s, 1H), 4.86 (s, 1H), 3.67 (s, 3H), 3.57 (s, 2H), 3.46 (s, 2H), 2.82 (qt, 2H), 2.69 (d, 2H), 2.48-2.39 (m, 6H), 2.30 (s, 3H)

### Example 3 Preparation of (S)-5-(1-(2-chlorophenyl)-2-methoxy-2-oxyethyl)-4,5,6,7-t etrahydrothieno[3,2-c]pyridin-2-yl-4-propylpiperazine-1-carboxylate (compound 3)

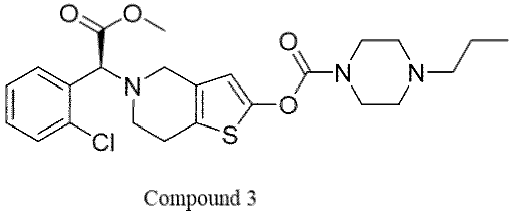

The preparation method was the same as that of Example 1, except that 4-methylpiperazine-1-formyl chloride hydrochloride was replaced with equimolar quantities of 4-propylpiperazine-1-formyl chloride hydrochloride to obtain the title compound, with a yield of 76.3% and a purity of 97.10%.
ESI-MS: m/z = 492.2(M+H)⁺
¹HNMR (400 MHz, DMSO-d6) δ: 7.58-7.44 (m, 1H), 7.45-7.36 (m, 1H), 7.31-7.22 (m, 2H), 6.29 (s, 1H), 4.75 (d, 1H), 3.90 (d, 2H), 3.71 (s, 3H), 3.40 (m, 4H), 3.15-3.08 (m, 1H), 3.01 (d, 1H), 2.91 (t, 2H), 2.48-2.39 (m, 6H), 1.57 (qt, 2H), 0.88 (t, 3H)

### Example 4: Preparation of (S)-5-(1-(2-chlorophenyl)-2-methoxy-2-oxyethyl)-4,5,6,7-tetrahydrothieno[3,2-c]pyridin-2-yl 4-cyclopropylpiperazine-1-carboxylate (compound 4)

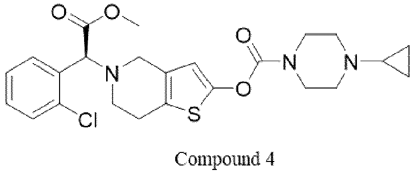

The preparation method was the same as that of Example 1, except that 4-methylpiperazine-1-formyl chloride hydrochloride was replaced with equimolar quantities of 4-cyclopropylpiperazine-1-formyl chloride hydrochloride to obtain the title compound, with a yield of 81.3% and a purity of 97.31%.
ESI-MS: m/z = 490.1(M+H)⁺
¹HNMR (400 MHz, DMSO-d6) δ: 7.52-7.43 (m, 1H), 7.43-7.34 (m, 1H), 7.32-7.21 (m, 2H), 6.29 (s, 1H), 4.75 (d, 1H), 3.90 (d, 2H), 3.71 (s, 2H), 3.45 (dd, 2H), 3.35 (dd, 2H), 3.15-3.08 (m, 1H), 3.02 (t, 1H), 2.91 (t, 2H), 2.74 (m, 2H), 2.61 (m, 2H), 2.53-2.45 (m, 1H), 0.77-0.50 (m, 4H)

### Example 5: Preparation of (S)-5-(1-(2-chlorophenyl)-2-methoxy-2-oxyethyl)-4,5,6,7-tetrahydrothieno[3,2-c]pyridin-2-yl 4-cyclopentylpiperazine-1-carboxylate (compound 5)

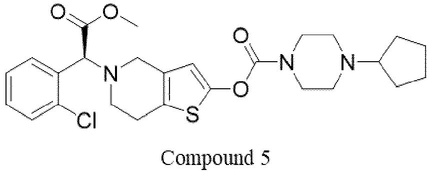

The preparation method was the same as that of Example 1, except that 4-methylpiperazine-1-formyl chloride hydrochloride was replaced with equimolar quantities of 4-cyclopentylpiperazine-1-formyl chloride hydrochloride to obtain the title compound, with a yield of 84.3% and a purity of 98.11%.
ESI-MS: m/z = 518.2(M+H)⁺
¹HNMR (400 MHz, DMSO-d6) δ: 7.66-7.51 (m, 1H), 7.46-7.38(m, 1H), 7.33-7.22 (m, 2H), 6.29 (s, 1H), 4.75 (d, 1H), 3.90 (d, 2H), 3.71 (s, 3H), 3.45 (dd, 2H), 3.35 (dd, 2H), 3.15-3.08 (m, 1H), 3.02 (t, 1H), 2.91 (t, 2H), 2.73 (m, 2H), 2.67-2.58 (m, 2H), 2.50 (pent, 1H), 1.88-1.74 (m, 4H), 1.64-1.45 (m, 4H)

### Example 6: Preparation of (S)-5-(1-(2-chlorophenyl)-2-methoxy-2-oxyethyl)-4,5,6,7-tetrahydrothieno[3,2-c]pyridin-2-yl 4-cyclohexylpiperazine-1-carboxylate (compound 6)

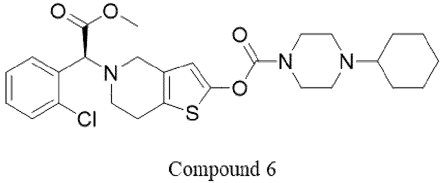

The preparation method was the same as that of Example 1, except that 4-methylpiperazine-1-formyl chloride hydrochloride was replaced with equimolar quantities of 4-cyclohexylpiperazine-1-formyl chloride hydrochloride to obtain the title compound, with a yield of 71.4% and a purity of 98.23%.
ESI-MS: m/z = 532.2(M+H)⁺
¹HNMR (400 MHz, DMSO-d6) δ: 7.62-7.50 (m, 1H), 7.46-7.35 (m, 1H), 7.30-7.18 (m, 2H), 6.29 (s, 1H), 4.75 (d, 1H), 3.88 (d, 2H), 3.71 (s, 3H), 3.45 (dd, 2H), 3.38 (dd, 2H), 3.18-3.08 (m, 1H), 3.02 (t, 1H), 2.91 (t, 2H), 2.73 (m, 2H), 2.67-2.58 (m, 2H), 2.34 (pent, 1H), 1.73-1.58 (m, 4H), 1.62-1.49 (m, 2H), 1.53-1.39 (m, 1H), 1.42-1.32 (m, 1H)

### Example 7: Preparation of (S)-5-(1-(2-chlorophenyl)-2-methoxy-2-oxyethyl)-4,5,6,7-tetrahydrothieno[3,2-c]pyridin-2-yl[1,4'-bipiperidine]-1'-carboxylate (compound 7)

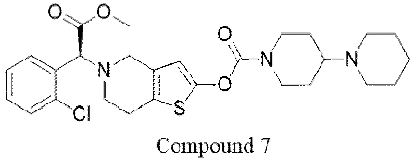

The preparation method was the same as that of Example 1, except that 4-methylpiperazine-1-formyl chloride hydrochloride was replaced with equimolar quantities of 4-piperidinyl piperidine formyl chloride hydrochloride to obtain the title compound, with a yield of 88.7% and a purity of 98.45%.
ESI-MS: m/z = 532.2(M+H)⁺
¹HNMR (400 MHz, DMSO-d6) δ: 7.52-7.43 (m, 1H), 7.43-7.34 (m, 1H), 7.32-7.21 (m, 2H), 6.29 (s, 1H), 4.75 (d, 1H), 3.90 (d, 2H), 3.71 (s, 3H), 3.69-3.59 (m, 2H), 3.39 (m, 2H), 3.15-3.08 (m, 1H), 3.02 (t, 1H), 2.91 (t, 2H), 2.68 (m, 2H), 2.39 (q, 1H), 2.31 (m, 2H), 1.74-1.37 (m, 10H)_{∘}

### Example 8: Preparation of (S)-5-(1-(2-chlorophenyl)-2-methoxy-2-oxyethyl)-4,5,6,7-tetrahydrothieno[3,2-c]pyridin-2-yl[1,4'-bipiperidine]-1'-carboxylate hydrochloride (compound 8)

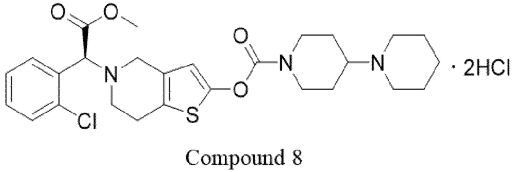

The preparation method was the same as that of Example 2, except that (S)-5-(1-(2-chlorophenyl)-2-methoxy-2-oxyethyl)-4,5,6,7-tetrahydrothieno[3,2-c]pyridin-2-yl 4-methylpiperazine-1-carboxylate was replaced with equimolar quantities of (S)-5-(1-(2-chlorophenyl)-2-methoxy-2-oxyethyl)-4,5,6,7-tetrahydrothieno[3,2-c]pyridin-2-yl[1,4 '-bipiperidin]-1'-carboxylate, and fumaric acid was replaced with equimolar quantities of HCl (ethyl acetate solution) to obtain the title compound, with a yield of 85.9% and a purity of 98.69%.
ESI-MS: m/z = 532.2(M+H)⁺
¹HNMR (400 MHz, DMSO-d6) δ: 7.58-7.46 (m, 1H), 7.46-7.34 (m, 1H), 7.39-7.24 (m, 2H), 6.29 (s, 1H), 4.75 (d, 1H), 3.90 (d, 2H), 3.71 (s, 3H), 3.71-3.60 (m, 2H), 3.35 (m, 2H), 3.15-3.08 (m, 1H), 3.02 (t, 1H), 2.91 (t, 2H), 2.68 (m, 2H), 2.39 (q, 1H), 2.31 (m, 2H), 1.74-1.37 (m, 10H)

### Example 9: Preparation of 5-((S)-1-(2-chlorophenyl)-2-methoxy-2-oxyethyl)-4,5,6,7-tetrahydrothieno[3,2-c]pyridin-2-yl-4-(1-methylpyrrolidine-3-yl)piperidine-1-carboxylate (comp ound 9)

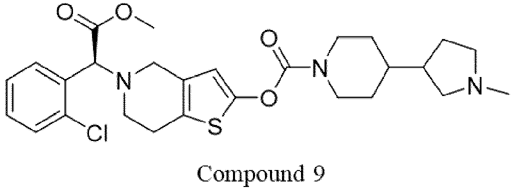

The preparation method was the same as that of Example 1, except that 4-methylpiperazine-1-formyl chloride hydrochloride was replaced with equimolar quantities of 4-(1-methylpyrrolidine-3-yl)piperidine formyl chloride hydrochloride to obtain the title compound, with a yield of 74.7% and a purity of 97.79%.
ESI-MS: m/z = 532.2(M+H)⁺
¹HNMR (400 MHz, DMSO-d6) δ: 7.82-7.65 (m, 1H), 7.55-7.44 (m, 1H), 7.38-7.25 (m, 2H), 6.29 (s, 1H), 4.75 (d, 1H), 3.90 (d, 2H), 3.73-3.59 (m, 5H), 3.34-3.22 (m, 2H), 3.15-3.08 (m, 1H), 3.02 (t, 1H), 2.91 (t, 2H), 2.74-2.52 (m, 4H), 2.23 (s, 3H), 2.13 (m, 1H), 1.86-1.75 (m, 2H), 1.79-1.70 (m, 2H), 1.73-1.63 (m, 1H), 1.67-1.60 (m, 1H), 1.63-1.55 (m, 1H), 1.58-1.47 (m, 1H)

### Example 10: Preparation of (S)-5-(1-(2-chlorophenyl)-2-methoxy-2-oxyethyl)-4,5,6,7 -tetrahydrothieno[3,2-c]pyridin-2-yl-4-(4-methylpiperazine-1-yl)piperidine-1-carboxylate (comp ound 10)

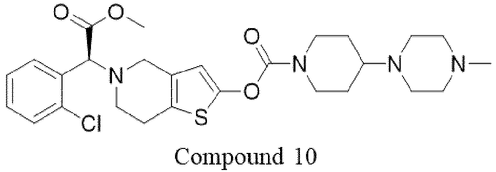

The preparation method was the same as that of Example 1, except that 4-methylpiperazine-1-formyl chloride hydrochloride was replaced with equimolar quantities of 4-(4-methylpiperazine-1-yl)piperidine formyl chloride hydrochloride to obtain the title compound, with a yield of 68.5% and a purity of 98.08%.
ESI-MS: m/z = 547.2(M+H)⁺
¹HNMR (400 MHz, DMSO-d6) δ: 7.88-7.66 (m, 1H), 7.53-7.45 (m, 1H), 7.36-7.23 (m, 2H), 6.33(s, 1H), 4.75 (d, 1H), 3.90 (d, 2H), 3.71 (s, 3H), 3.65 (m, 2H), 3.39 (m, 2H), 3.15-3.08 (m, 1H), 3.02 (t, 1H), 2.91 (t, 2H), 2.68-2.58 (m, 1H), 2.62-2.54 (m, 3H), 2.57-2.50 (m, 2H), 2.54-2.45 (m, 3H), 2.40 (pent, 1H), 2.33 (s, 3H), 1.74-1.56 (m, 4H)

### Comparative Example 1: 2-Oxo-clopidogrel (intermediate metabolite during clopidogrel activation)

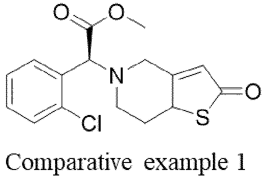

It was prepared by Chengdu Shibeikang Biomedical Technology Co., Ltd, ee=98.8%.

### Comparative Example 2: Vicagrel (in clinical study)

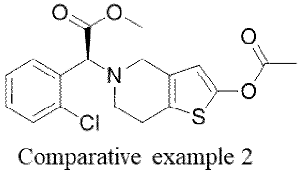

It was prepared according to CN201010624329.7, with a purity of 98.22%.

### Test Example 1 Study on antiplatelet aggregation effect

1. Test objective: Evaluation and comparison of the antiplatelet aggregation treatment effect of each compound after administration at the same molar amount
2. Test method

### (1) Grouping

70 rats were randomly and evenly grouped into 7 groups, i.e., solvent control group, comparative example 1 (2-Oxo-clopidogrel) group, comparative example 2 (Vicagrel) group, compound 1 group, compound 2 group, compound 7 group and compound 8 group.

### (2) Induction of anesthesia

Rats were anesthetized by intraperitoneal injection of 1.5% sodium pentobarbital.

### (3) Fixation

The anesthetized animals were transferred to an operating platform. The rats were observed for eyelid reflex and response to pain stimulation to ensure that the eyelid reflex and response of the limbs and tail to pain stimulation had disappeared before surgery began.

### (4) Blood collection from the abdominal aorta of rat

The rats were injected with anesthetic, and fixed on the operating platform in a supine position after the whole body of the rat became soft. After routine disinfection, the abdomen of the rat was incised along the abdominal midline using surgical scissors to open the abdominal cavity. The adipose tissue around the blood vessels was gently separated with a small forceps, and the excess adipose tissue covering the blood vessels was then wiped and removed with the cotton ball to make the blood vessels clearly visible (the abdominal aorta is located anterior to the vertebral column, and the abdominal vein appears thicker and darker in color than the abdominal aorta). The blood vessel was first fixed to prevent it from moving as much as possible. The left thumb and index finger fixed the adipose tissue and other organs on both sides of the blood vessel, while the ring finger pressed on the blood vessel at the upper end of the needle insertion point to lower the blood pressure, which can prevent blood spurting. The puncture needle was held with the right hand, with the bevel of the needle tip facing downward. The needle was inserted at an angle of about 30 degrees and advanced towards the heart to a suitable depth of about 5 mm. Once the blood had started to enter the needle via its tip, the other end of the blood collection needle was inserted into the vacuum tube. After the insertion of needle, the needle tip could be clamped with a hemostatic clip to prevent it from puncturing the blood vessel if the rat struggled due to insufficient anesthesia.

### (5) Preparation of platelet

Three hours after the administration, the rats were anesthetized with sodium pentobarbital. Then blood was collected from the abdominal aorta, added with 3.8% sodium citrate (1:9) as an anticoagulant, mixed evenly and centrifuged at 200 g for 10 min. The supernatant was harvested to obtain the platelet-rich plasma (PRP), and the residual plasma was then centrifuged at 1600 g for 15 min to obtain the resulting supernatant as platelet-poor plasma (PPP).

### (6) Induction of platelet aggregation by ADP

Platelet aggregation rate was measured using a platelet aggregometer (model: Agg RAM, Helena, USA). The PPPs corresponding to the PRPs to be tested in each channel were first used to correct the light transmission, and the PPPs were removed after the correction. A cuvette added with 225 µl of PRP to be tested was then placed in each channel, and added with a stirrer, then ADP (with a final concentration of 20 µM). The platelet aggregometer immediately began to measure the platelet aggregation rate.

### 3. Test results

After the administration at the same molar amount, data of platelet aggregation rate of each group is detailed in Table 1.

The results show that (1) the platelet aggregation rate (%) of the solvent control group was 76.06±3.29, the platelet aggregation rates (%) of the comparative example 1 group, comparative example 2 group, compound 1 group, compound 2 group, compound 7 group and compound 8 group were 31.32±6.07, 39.32±12.38, 24.08±6.00, 21.18±8.51, 27.59±6.99 and 27.13±6.50, respectively. Compared to the solvent control group, all administration groups showed significant inhibition effect on ADP-induced platelet aggregation in rats (all P<0.01).

(2) The compound 1, compound 2, compound 7, and compound 8 groups had a significant advantage in inhibiting ADP-induced platelet aggregation in rats compared to the comparative example 2 group, showing a statistical significance (for compound 1 and compound 2 groups, P<0.01; for compound 7 and compound 8 groups, P<0.05).

(3) As can be seen from the test results, after the administration of single intravenous injection at the same dose, the inhibition effect of the compound 1, compound 2, compound 7 and compound 8 of the present disclosure on ADP-induced platelet aggregation in rats was significantly better than that of comparative example 2 compound, and also better than that of comparative example 1 compound.

**Table 1 Results of antiplatelet aggregation efficacy assay of each group after administration at the same molar dose**

| Group | Route of administration | Dose (mg/kg) | Number of animals (male) | Platelet aggregation rate (%) |
|---|---|---|---|---|
| Solvent control group | iv | \ | 10 | 76.06±3.29 |
| Comparative Example 1 group | iv | 3.00 | 10 | 31.32±6.07** |
| Comparative Example 2 group | iv | 3.37 | 10 | 39.32±12.38** |
| Compound 1 group | iv | 4.12 | 10 | 24.08±6.00**^{▲▲} |
| Compound 2 group | iv | 6.18 | 10 | 21.18±8.51**^{▲▲} |
| Compound 7 group | iv | 4.71 | 10 | 27.59±6.99**^{▲} |
| Compound 8 group | iv | 5.36 | 10 | 27.13±6.50**^{▲} |

| | | | | |
|---|---|---|---|---|
| Note: ** represents P<0.01 compared to solvent control group; and A A and A represent P<0.01 and P<0.05, respectively, compared to comparative example 2 group. | | | | |

### Test Example 2: Effect of compounds on current in stably overexpressed hERG channel

### 1. Test samples

Compounds 1 to 10 and the control compound Cisapride

### 2. Test methods

The inhibition effect of the test compounds 1 to 10 on the hERG potassium channel was investigated by manual patch-clamp technique (the gold standard for hERG safety evaluation) to evaluate their risk of triggering ventricular repolarization toxicity.

### 3. Test results

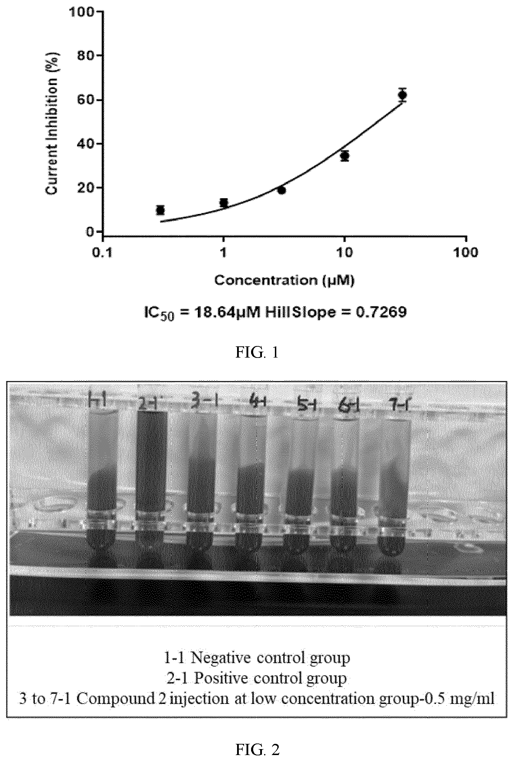

In this study, the concentration-effect relationship of the test compounds 1-10 on the blockade of hERG channels was detected by the manual patch-clamp technique to evaluate the risk of the inhibitory effect of the test compounds on cardiac hERG potassium channels. Among them, the concentration-effect curve of the compound 2 on current is shown in FIG. 1, and the test results are shown in Table 2.

**Table 2 Results of inhibition effect of test compounds on cardiac hERG potassium channel**

| Test sample | IC₅₀ (µM) | Test sample | IC₅₀ (µM) |
|---|---|---|---|
| Compound 1 | 20.50 | Compound 2 | 18.64 |
| Compound 3 | 15.16 | Compound 4 | 16.41 |
| Compound 5 | 15.78 | Compound 6 | 14.90 |
| Compound 7 | 25.43 | Compound 8 | 27.33 |
| Compound 9 | 27.18 | Compound 10 | 25.95 |
| Cisapride | 15.81 | | |

| | | | |
|---|---|---|---|
| Note: The IC₅₀ value of the positive drug Cisapride above is presented in a unit of nM. | | | |

The above test results show that the IC₅₀ value of the positive drug was less than 0.1 µM, indicating that the positive drug has cardiac toxicity and the modeling of this test was successful. However, the IC₅₀ values of the compounds of the present disclosure were all greater than 10 µM, proving that the compounds of the present disclosure had a weakly inhibitory or no inhibitory effect on hERG, which means that the compounds of the present disclosure have a small risk of triggering ventricular repolarization toxicity, and are highly safe.

### Test Example 3 Pharmacokinetic study

### 1. Test objective

After being administered via single gavage at the same molar amount, the pharmacokinetic characteristics of each compound were investigated, and the main pharmacokinetic parameters were compared.

### 2. Materials and methods

### (1) Test samples

Comparative example 1 (2-Oxo-clopidogrel), compound 1, compound 2, compound 7 and compound 8.

### (2) Preparation of formulation for administration

Each test compound was accurately weighed separately into a clean drug container, dissolved with appropriate amount of Solutol, shaken by vortex, added with purified water, ultrasonicated, and shaken by vortex until the compound was completely dissolved. All the formulations were freshly prepared on the day of administration.

### (3) Test grouping and administration regimen

Thirty healthy adult SD rats were administered via gavage. The specific scheme is shown in Table 3.

**Table 3 Administration regimen in pharmacokinetic assay of rats**

| Test sample | Number of animals | Administration dose (mg/kg) | Administration volume (ml/kg) | Route of administration |
|---|---|---|---|---|
| Comparative example 1 | 6 | 3.00 | 10 | iv. |
| Compound 1 | 6 | 4.12 | 10 | iv. |
| Compound 2 | 6 | 6.18 | 10 | iv. |
| Compound 7 | 6 | 4.71 | 10 | iv. |
| Compound 8 | 6 | 5.36 | 10 | iv. |

### (4) Test methods

Grouping and Fasting: SD rats were randomly grouped, with 6 rats per group, and administered with the corresponding compounds via gavage according to Table 3.

Sample Collection and Processing: 0.2 ml of blood was collected at different time points of before (0 h) and 5 min, 15 min, 30 min, 1 h, 2 h, 4 h, 8 h, and 24 h after the administration. After the anticoagulation with EDTA-K2 was completed, the blood was centrifuged at 4°C for 5 min, and then the plasma was separated and stored at -80°C for later measurement.

Measurement: The blood drug concentration of 2-Oxo-clopidogrel in all PK plasma samples was measured by LC/MS/MS method, and the pharmacokinetic parameters of individual compounds were calculated by using software WinNonlin 7.2.

### 3. Test results

**Table 4 Main pharmacokinetic parameters of 2-Oxo-clopidogrel in plasma of each compound (n=6, mean ± SD)**

| Test sample | Tₘₐₓ (h) | T_{1/2} (h) | Cₘₐₓ (ng/ml) | AUC₀₋ₗₐₛₜ (h*ng/ml) |
|---|---|---|---|---|
| Comparativ e example 1 | 0.63±0.15 | 1.18±0.21 | 38.48±13.51 | 60.15±14.22 |
| Compound 1 | 0.48±0.16 | 1.69±0.23 ** | 69.32±18.40** | 99.32±23.47** |
| Compound 2 | 0.45±0.13 | 1.98±0.25** | 76.82±21.69** | 110.15±32.62** |
| Compound 7 | 0.55±0.14 | 1.53±0.19* | 60.98±15.03 * | 90.98±30.45* |
| Compound 8 | 0.58±0.13 | 1.47±0.22* | 57.65±13.64* | 89.32±23.27* |

| | | | | |
|---|---|---|---|---|
| Note: ** represents P<0.01 and * represents P<0.05, compared to the comparative example 1 compound group. | | | | |

As shown in the table above, the T_{1/2}, Cₘₐₓ and AUC₀₋ₗₐₛₜ of the compound 1, compound 2, compound 7, and compound 8 were significantly prolonged and improved compared to the comparative example 1 compound, showing statistical significances. Among them, both the compounds 1 and 2 showed P<0.01, and both the compounds 7 and 8 showed P<0.05, indicating that the compounds 1, 2, 7 and 8 exhibited significantly better absorption in the body of rat and had better pharmacokinetic characteristics, compared to the comparative example 1 compound.

In summary, the compounds of the present disclosure have better absorption and high bioavailability, and are expected to exhibit complete therapeutic efficacy.

### Test Example 4: In vitro hemolysis test

1. Test sample
   Compound 2
2. Test animal
   One adult big white-eared rabbit (male)
3. Preparation of red blood cell suspension
   (1) After the rabbit was anesthetized by injection of sodium pentobarbital via the marginal ear vein at about 30 mg/kg, about 20 ml of blood was collected from the heart into an appropriate container.
   (2) A stirrer (or glass bead) was placed in the container, the blood was stirred at a slow speed for a few minutes to remove the fibrinogen in the blood so as to obtain defibrinated blood.
   (3) About 10 times the amount of physiological saline was added, mixed evenly by shaking and aliquoted into centrifuge tubes. The tubes were centrifuged at 1000 to 2000 rpm for 15 min (the rotational speed and time for centrifugation could be adjusted according to the post-centrifugation situation due to the difference of centrifuge tubes in size), and then the supernatants were removed.
   (4) The resulting precipitated red blood cells were then washed with physiological saline according to the method above again until the supernatant did not show red color.
   (5) The obtained red blood cells were prepared into a 2% suspension with physiological saline.
4. Formulations for hemolysis assay *in vitro*

**Table 5 Formulations for hemolysis assay in vitro**

| Group | Tube numbering | 2% red blood cell suspension (ml) | 0.9% sodium chloride injection (ml) | Test sample (ml) | Sterilized water for injection (ml) |
|---|---|---|---|---|---|
| Negative control group a | 1 | 2.5 | 2.5 | - | - |
| Positive control group b | 2 | 2.5 | - | - | 2.5 |
| Low concentration of compound 2 injection (0.5 mg/ml) | 3 | 2.5 | 2 | 0.5 | - |
| | 4 | 2.5 | 2.1 | 0.4 | - |
| | 5 | 2.5 | 2.2 | 0.3 | - |
| | 6 | 2.5 | 2.3 | 0.2 | - |
| | 7 | 2.5 | 2.4 | 0.1 | - |
| High concentration of compound 2 injection (2.5 mg/ml) | 8 | 2.5 | 2 | 0.5 | - |
| | 9 | 2.5 | 2.1 | 0.4 | - |
| | 10 | 2.5 | 2.2 | 0.3 | - |
| | 11 | 2.5 | 2.3 | 0.2 | - |
| | 12 | 2.5 | 2.4 | 0.1 | - |

| | | | | | |
|---|---|---|---|---|---|
| Note: The test sample of the negative control group^{a} above is a mixture of 2.5 ml of a 2% red blood cell suspension and 2.5 ml of a 0.9% sodium chloride injection, and the test sample of the positive control group^{b} is a mixture of 2.5 ml of a 2% red blood cell suspension and 2.5 ml of sterilized water for injection. | | | | | |

### 5. Determination criteria for results

**Table 6 Determination criteria for results of hemolysis assay**

| Indication | Determination criteria |
|---|---|
| Complete Hemolysis | Clear red solution, no red blood cells at the bottom of the tube |
| Partial Hemolysis | Clear red or brown solution, small amount of residual red blood cells at the bottom of the tube |
| No Hemolysis | All red blood cells sink, and the upper layer of liquid is colorless and clear. |
| True Agglutination | Red blood cells aggregate into clots that cannot be dispersed after shaking. |
| False Agglutination | Red blood cells aggregate into clots that can be uniformly dispersed after shaking. |

### 6. Test results

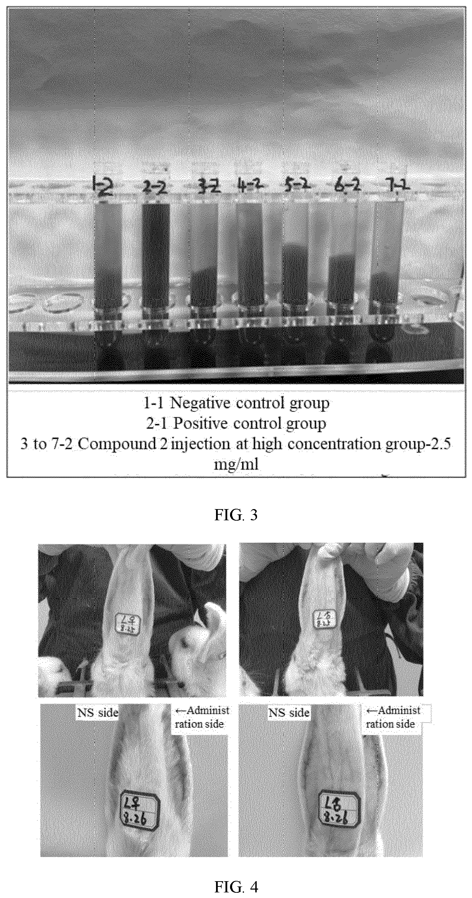

The test results are shown in FIGs. 2 and 3. According to the test results, it can be seen that neither the compound 2 injection at low concentration (0.5 mg/ml) nor the compound 2 injection at high concentration (2.5 mg/ml) caused hemolysis of rabbit blood cells.

### Test Example 5: Vascular irritation assay

1. Test sample
   Compound 2
2. Test animals
   Four Japanese big-ear white rabbit (about 2kg), 2♀/2♂ (2 female/2 male)
3. Test protocol

The intra-individual left/right comparison method was used. The compound 2 low dose injection and compound 2 high dose injection groups were set, with 2 rabbits in each group, half male and half female. The compound 2 injections were administered by injection into the ear marginal veins of right ears of the rabbits in the compound 2 low and high dose groups, with administration doses of 2.5 mg/kg and 7.5 mg/kg, respectively, and with the same administration volume of 5 ml/kg; and the corresponding administration concentrations were 0.5 mg/ml and 1.5 mg/ml, respectively. The same administration volume of sodium chloride injection as the negative control was administered by injection into the ear marginal veins of left ears of the rabbits in the compound 2 low and high dose groups. The administration was carried out by intravenous infusion for 30 min. After the administration was completed, the animals were observed for their general state, body weight, food intake, and local reactions around the injection site (including edema, congestion, degeneration, and necrosis). The administration was performed once daily for a total of 7 consecutive days.

At 72 h and at the end of a 14-day recovery period after the final administration, half of the animals in each group (1♀ and 1♂) were sacrificed, respectively. Marginal ear tissue samples were cut from the injection site and the proximal end of the marginal ear vein respectively and collected. The injection sites were observed by naked eyes for the response conditions to irritation. The obtained tissues were fixed with 10% neutral formalin and conventionally sectioned for histopathological examination.

Note: The specific administration time should be appropriately adjusted according to the situation after the administration.

### 4. Administration scheme

**Table 7 Administration scheme**

| Group | Number of animals (number ) | Administra tion dose (mg/kg) | Administr ation concentrat ion (mg/ml) | Administ ration volume (ml/kg) | Route of administrati on | solvent |
|---|---|---|---|---|---|---|
| compound 2-L group (female) | 1 | 2.5 | 0.50 | 5 | iv. (30 min) | 0.9% sodium chloride injection |
| compound 2-L group (male) | 1 | 2.5 | 0.50 | 5 | iv. (30 min) | |
| compound 2-H group (female) | 1 | 7.5 | 1.50 | 5 | iv. (30 min) | |
| compound 2-H group (male) | 1 | 7.5 | 1.50 | 5 | iv. (30 min) | |

### 5. Test results

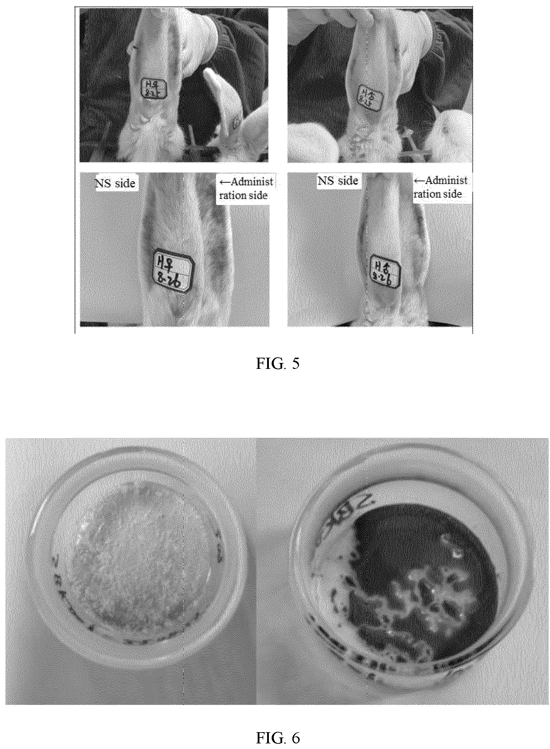

The test results are shown in the following table, FIGs. 4 and 5. The low concentration of compound 2 (0.5 mg/ml) had no vascular irritation effect on the ear marginal vein of the rabbit, as observed by naked eyes. In the compound 2 high concentration group (1.5 mg/ml), although the congestion of the ear marginal vein on the administered side was observed by naked eyes at day 2 of the recovery period, considering that this recovery period was short, the high concentration of compound 2 (1.5 mg/ml) was determined to have no vascular irritation effect on the ear marginal vein of the rabbit.

**Table 8 Results of vascular irritation assay**

| Group | Number of animal (number) | Administration concentration (mg/ml) | Route of administr ation | Day 1 to Day 7 (Administrati on period) | Day 8 to Day 9 (Recovery period) |
|---|---|---|---|---|---|
| compoun d 2-L group (female) | 1 | 0.5 | iv | No apparent abnormal response was observed by naked eyes | No congestion or oedema of the drug-administrated ear marginal vein and surrounding tissues at the injection site was observed by naked eyes |
| compoun d 2-L group (male) | 1 | 0.5 | iv | No apparent abnormal response was observed by naked eyes | No congestion or oedema of the drug-administrated ear marginal vein and surrounding tissues at the injection site was observed by naked eyes |
| compoun d 2-H group (female) | 1 | 1.5 | iv | No apparent abnormal response was observed by naked eyes | Congestion of the drug-administrated ear marginal vein and surrounding tissues at the injection site was observed by naked eyes |
| compoun d 2-H group (male) | 1 | 1.5 | iv | No apparent abnormal response was observed by naked eyes | No congestion or oedema of the drug-administrated ear marginal vein and surrounding tissues at the injection site was observed by naked eyes |

### Test Example 6: Solubility study

Samples of the example compound 2 and comparative example 2 (Vicagrel) were accurately weighed. Under the condition of 25°C to 30°C, 1 ml of physiological saline and 1 ml of a buffer (pH 1.2) were taken, and added with 10 mg of each compound, respectively. The measured solubility data are shown in the following table. The compound 2 of the present disclosure had excellent solubility, which can satisfy the demand for the formulation into an injection with an effective concentration in clinical, while the solubility of the comparative example 2 was extremely low and it cannot satisfy the demand for injection, and the addition of additional solubilizer may pose potential safety risks.

**Table 9 Results of solubility test**

| Sample | Physiological saline(1 ml) | pH 1.2 buffer |
|---|---|---|
| Compound 2 | >10 mg | >10 mg |
| Comparative example 2 | insoluble | <0.5 mg |

### Test Example 7: Influencing factor test

Test Methods: The samples of the compound 2 and comparative example 2 (Vicagrel) were weighed and placed in weighing bottles, respectively, and the weighing bottles were placed under the conditions of high temperature (60°C), high humidity (RH 80%), and light (5000 Lux), respectively.

The test results of the compound 2 are shown in the table below. After being placed under each of the following conditions for 10 days: high temperature, high humidity and light, the compound 2 showed little change in content and no obvious change in appearance and shape, indicating good stability.

**Table 10 Results of influencing factor test**

| Sample | Day 0 | High temperature (Day 10) | High humidity (Day 10) | Light (Day 10) |
|---|---|---|---|---|
| Compound 2 | 98.39% | 97.84% | 97.83% | 96.09% |

In contrast, after being placed under high temperature (60°C) for two days, the comparative example 2 showed significant changes in the appearance and characteristics, from a white solid to a yellowish-brown oil, indicating that the comparative example 2 has a poor stability under the high temperature condition. The appearance and characteristics of the comparative example 2 at Day 0 and after being placed under the high temperature (60°C) for 2 days is shown in FIG. 6.

In summary, the compounds of the present disclosure have better stability against temperature, humidity and light compared to the comparative example 2.

Various modifications and variations to the compounds, compositions, and methods of the present disclosure can be made by those of ordinary skill in the art without deviating from the spirit of the present disclosure, and these modifications and variations fall within the same or equivalent scope as the present disclosure.

## Claims

1. A compound represented by Formula (I), or a pharmaceutically acceptable salt, solvate or deuteride thereof: 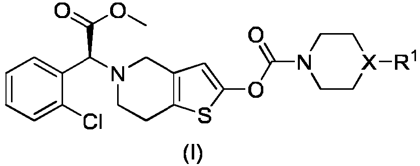 wherein,
X is selected from the group consisting of C or N, and
R¹ is selected from the group consisting of substituted or unsubstituted alkyl, substituted or unsubstituted cycloalkyl, or substituted or unsubstituted N-containing heterocyclyl.

2. The compound according to claim 1, or the pharmaceutically acceptable salt, solvate or deuteride thereof, wherein,
X is selected from the group consisting of C or N, and
R¹ is selected from the group consisting of substituted or unsubstituted C₁ to C₆ alkyl, substituted or unsubstituted C₃ to C₆ cycloalkyl, or substituted or unsubstituted C₂ to C₆ N-containing heterocyclyl.

3. The compound according to claim 2, or the pharmaceutically acceptable salt, solvate or deuteride thereof, wherein,
X is N,
R¹ is selected from the group consisting of substituted or unsubstituted C₁ to C₆ alkyl, or substituted or unsubstituted C₃ to C₆ cycloalkyl; preferably, R¹ is selected from the group consisting of methyl, ethyl, propyl, cyclopropyl, cyclopentyl or cyclohexyl.

4. The compound according to claim 2, or the pharmaceutically acceptable salt, solvate or deuteride thereof, wherein,
X is C,
R¹ is selected from the group consisting of substituted or unsubstituted C₂ to C₆ N-containing heterocyclyl; preferably, R¹ is selected from the group consisting of piperazinyl, piperidinyl or pyrrolyl.

5. The compound according to claim 2, or the pharmaceutically acceptable salt, solvate or deuteride thereof, wherein the compound is selected from the group consisting of:

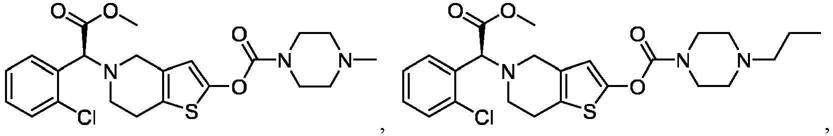

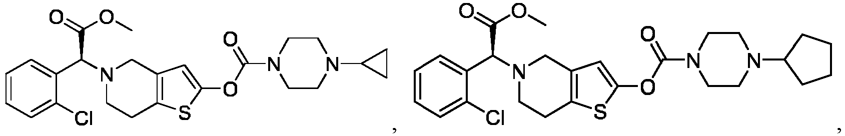

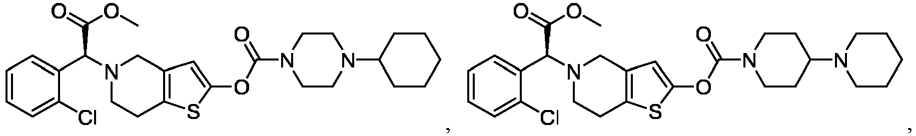

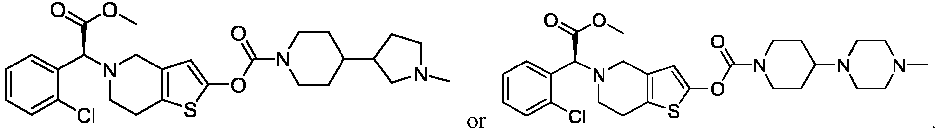

6. The compound according to any one of claims 1 to 5, or the pharmaceutically acceptable salt, solvate or deuteride thereof, wherein the pharmaceutically acceptable salt includes fumarate, acetate, ascorbate, benzoate, benzenesulfonate, citrate, hydrochloride, hydrobromide, maleate, mesylate, sulfate, hydrosulfate, nitrate, oxalate, phosphate or succinate.

7. The compound according to claim 6, or the pharmaceutically acceptable salt, solvate or deuteride thereof, wherein the compound or the pharmaceutically acceptable salt thereof is selected from the group consisting of: 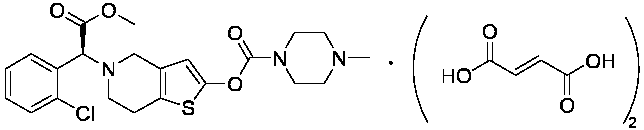 or

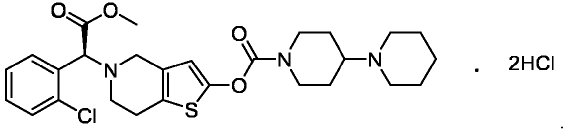

8. A method for producing the compound according to any one of claims 1 to 7, or the pharmaceutically acceptable salt, solvate or deuteride thereof, comprising: 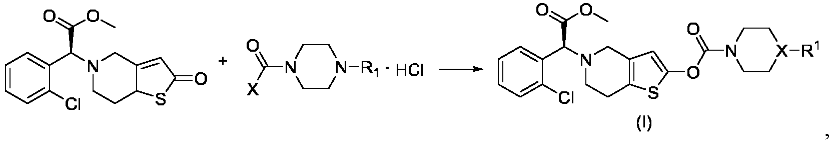 wherein, R¹ is the same as defined in any one of claims 1 to 7, and X is halogen.

9. Use of the compound according to any one of claims 1 to 7, or the pharmaceutically acceptable salt, solvate or deuteride thereof in the manufacture of a medicament for preventing and/or treating a cardiovascular, cerebrovascular or other arterial circulatory disease due to platelet aggregation.

10. The use according to claim 9, wherein the cardiovascular, cerebrovascular or other arterial circulatory disease due to platelet aggregation includes, but is not limited to, acute coronary artery syndrome, atherosclerotic disease or thrombotic complication.
